# EUROPÄISCHE PATENTSCHRIFT

(11) **EP 2 708 264 B1**
(45) Veröffentlichungstag und Bekanntmachung des Hinweises auf die Patenterteilung: **04.10.2017**
(21) Anmeldenummer: 13175941.7
(22) Anmeldetag: 10.07.2013
(51) Int. Cl.: A61Q 19/08, A61K 8/14, A61K 8/73, A61Q 19/00, A61K 8/35, A61K 8/55, A61K 8/69, A61K 8/11, A61K 8/97

(54) **Kosmetisches Zweikomponenten-Präparat zur getrennten Lagerung von Zubereitungen enthaltend Liposomen enthaltend Coenzym Q10 bzw. Fluorocarbone**
Cosmetic two component preparation for separate storage of compositions comprising liposomes of coenzyme Q10 and fluorocarbons
Préparation cosmétique bicomposant pour le stockage séparé des compositions comprenant des liposomes de coenzyme Q10, ainsi que de fluorocarbones

(30) Priorität: 13.09.2012 EP 12184281
(43) Veröffentlichungstag der Anmeldung: 19.03.2014
(73) Patentinhaber: PM-International AG, 5445 Schengen (LU)
(72) Erfinder: Golz-Berner, Karin, MC-98000 Monaco (MC); Sorg, Rolf, L-5444 Schengen (LU); Meinhardt, Horst, 56249 Herschbach (DE)
(74) Vertreter: Gulde & Partner

(56) Entgegenhaltungen:
- EP-A1- 0 509 338
- EP-A1- 0 611 207
- WO-A1-94/00098
- WO-A1-2005/016309
- WO-A2-02/060394
- DE-A1- 4 327 679
- JP-A- 2005 330 257
- JP-A- 2006 137 684
- Rita corporation: "General Product Listing", , 1. Januar 2006 (2006-01-01), XP007921622, Gefunden im Internet: URL:http://www.ritacorp.com/files/Domestic _GPL.pdf [gefunden am 2013-03-05]
- Rita Corporation: "ANTI-AGING OXYGEN TREATMENT", Personal Care - Ingredients - Formulation - Manufacture , 5. März 2013 (2013-03-05), XP002693509, Gefunden im Internet: URL:http://www.personalcaremagazine.com/Fo rmulationDetails.aspx?Formulation=290 [gefunden am 2013-03-05]
- DATABASE GNPD [Online] MINTEL; 1. Juli 2007 (2007-07-01), Skyn Iceland: "Hydro Cool Firming Eye Gel", XP002693510, Database accession no. 745624
- "NataPres MSDS", 20100801, Nr. 30-4399, 1. August 2010 (2010-08-01), Seiten 1-83, XP007921621,

## Beschreibung

Die Erfindung betrifft ein kosmetisches Zweikomponenten-Präparat mit verstärkter Anti-Alterungswirkung für die Haut.

Der Wirkstoff Ubichinon-10 oder Coenzym Q10 (Q-10) ist seit Jahren bekannt und viel genutzt in der kosmetischen Industrie. Q-10 verringert Hautschädigungen, da es freie Radikale wirksam verringert und damit Alterungsprozesse in der Haut aufschiebt. Bekannt ist auch die Verarbeitung von Q-10 in kosmetischen Cremes und Lotionen unterschiedlicher Art. Dabei sind jedoch Konzentrationen unter 10 % Q-10 handelsüblich, da bei höheren Konzentrationen schon nach wenigen Wochen Lagerungszeit Entmischungseffekte in den Emulsionen auftreten.

In der DE 10 2010 63 585 A1 wird beispielsweise in [0140] der Anteil an Coenzym Q10 für den Bereich von 0,0001 bis 1 Gew.-% angegeben, vorzugsweise bis 0,1 Gew.-%.

Es sind weiterhin Produkte auf dem Markt, in denen Q-10 kristallin in festen Kapseln angeboten wird, z. B. 100 mg pro Kapsel.

Auch für den Einsatz von Fluorcarbonen in der Kosmetik sind eine Reihe von Patenten bekannt, unter anderem die WO 94/00098, die WO 02/060394, die DE 4327679 A1 oder die WO 2005/016309. Darin wird teilweise auch ein Ver dickungsmittel in den Beispielen erwähnt, jedoch beschäftigen sich die Patente nicht mit der Stabilisierung der Sauerstoffvesikel sondern mit dem Transport von 0₂ in das Stratum corneum und tiefer.

Der Erfindung liegt die Aufgabe zugrunde, ein topisches kosmetisches Präparat mit einem sehr hohen Q-10-Anteil bereitzustellen, bei dem das Problem der Lagerstabilität eines Gemisches mit Q-10 nicht relevant ist.

Eine weitere Aufgabe besteht darin, ein topisches kosmetisches Präparat bereitzustellen, das für alternde Haut zu einer schnellen Hautregenerierung und zu einem verbesserten Erscheinungsbild der Haut führt.

Eine weitere Aufgabe besteht darin, ein topisches kosmetisches Präparat bereitzustellen, bei dem die darin enthaltenen Fluorcarbonliposomen, die Sauerstoff enthalten, diesen Sauerstoff für einen längeren Zeitraum in Kontakt mit der Haut halten.

Die Aufgabe wird erfindungsgemäß gelöst mit einem kosmetischen Zweikomponenten-Präparat gemäß den Ansprüchen. Das kosmetische Zweikomponenten-Präparat ist dadurch gekennzeichnet, daß es enthält
20 bis 40 Gew-% einer ersten in Liposomen verkapselten Substanz auf Basis von Ubichinon-10 und
60 bis 80 Gew-% eines wäßrigen, gelartigen Gemisches, wobei das Gemisch
a) eine erste Gruppe verdickender Substanzen, ausgewählt unter Gellan Gum, Carbomer, Xanthan Gum, Guar Gum und Gemischen davon,
b) eine zweite Gruppe verdickender Substanzen, ausgewählt unter Gummiarabicum, Tragenth, verschiedenen Cellulosen, Pektin, Magnesium-Aluminium-Silicaten, einem Pflanzenextraktgemisch aus Leuconostoc/Radish Root Ferment Filtrate, Lonicera Japonica Flower Extract, Lonicera Caprifolium Flower Extract und Populus Tremuloides Bark Extrakt, sowie Gemischen davon,
c) eine dritte Gruppe verdickender Substanzen, ausgewählt unter Stärken, wobei das Verhältnis der ersten, zweiten und dritten Gruppe verdickender Substanzen im Bereich von 1 : 6 - 40 : 6 - 60 liegt,
d) einen einwertigen C₂-C₄-Alkohol und
e) eine zweite in Liposomen verkapselten Substanz auf Basis von Fluorcarbonen umfaßt,
und die Viskosität nach Brookfield der ersten verkapselten Substanz und des wäßrigen gelartigen Gemisches jeweils im Bereich von 1000 bis 3000 mPa·s liegt, gemessen bei 25°C, Tellerspindel RV3,
und wobei die erste verkapselte Substanz in einem von dem gelartigen Gemisch getrennten ersten Container vorliegt und das gelartige Gemisch in einem zweiten Container, und wobei die Konzentrationsangaben auf das Gesamtgewicht des Präparats bezogen sind,
hergestellt durch Verrühren der ersten Gruppe verdickender Substanzen mit Wasser bei 50-70°C, danach Abkühlen auf 30-45°C und Zugabe der zweiten und dritten Gruppe verdickender Substanzen und gegebenenfalls kosmetischer Hilfsstoffe und anschließendes Homogenisieren bei höchstens 1500 U/min für 10 bis 15 Minuten zur Bildung eines Verdickerkomplexes, Abkühlen des Verdickerkomplexes auf 20-30°C, Zugabe des Alkohols und der zweiten verkapselten Substanz und homogenes Verrühren bei 200-300 U/min und Lagerung des Präparates bei 12-20°C.

Es wurde gefunden, daß bei dem späteren Vermischen der Q-10 enthaltenden ersten verkapselten Substanz und dem gelartigen Gemisch nach Öffnung beider Container vom Anwender weitgehend genau definierbare Mengen miteinander z.B. durch Schütteln in Kontakt gebracht werden und diese ein homogenes Gel bilden. Da diese definierte Menge über einen Zeitraum von etwa 2 - 12 Wochen lagerungsstabil ist, kann selbst nach monatelanger Lagerung des Zweikomponentenpräparats die vom Anwender benötigte Menge selbst bestimmt werden und der darin enthaltene hohe Q-10-Gehalt mit einem homogenen topisch anwendbaren, kosmetischen Präparat zum gewünschten Zeitpunkt realisiert werden. In diesem Zeitraum treten keine Entmischungserscheinungen des erfindungsgemäßen Präparats auf.

Ein bevorzugter Zeitraum ohne Entmischung des Gemisches aus dem ersten und dem zweiten Container liegt bei 3 - 10 Wochen, insbesondere 4 - 9 Wochen. In dieser Zeit sollte das vermischte Produkt bei maximal 15 °C oder vorzugsweise im Kühlschrank gelagert werden.

Ohne an eine Theorie gebunden zu sein, wird vermutlich die Lagerstabilität auch durch den relativ geringen Energieeintrag beim Vermischen z. B. Schütteln der beiden Containerinhalte gefördert.

Das wäßrige Gemisch mit den verdickenden Substanzen wird als "gelartig" bezeichnet, da der typische Hydrogelcharakter sowohl durch Stärke als auch durch den Alkoholgehalt modifiziert wird. Der Alkohol ist jedoch erforderlich, weil damit die dünnflüssige Erscheinung des Gels im Verhältnis zu den verdickenden Substanzen erhalten bleibt und das Auftragen des Gels auf die Haut durch den Anwender zu einem sehr angenehmen Hautgefühl führt. Ein solches "Hautfeeling" ist in der modernen Kosmetik ein beachtlicher Auswahlfaktor von Produkten für den Anwender geworden. Dieses "Hautfeeling" über den Alkohol bei Vorhandensein der anderen Komponenten steuern zu können, ist durchaus überraschend.

Der Gehalt eines bevorzugten Alkohols wie Ethanol oder i-Propanol liegt vorteilhaft im Bereich von 3-10 Gew.-%, bezogen auf das Gesamtgewicht des kosmetischen Präparates, vorzugsweise im Bereich von 4 bis 7 Gew.-%, insbesondere 5 bis 7 Gew.-%. Der bevorzugte Alkohol ist Ethanol.

Eine bevorzugte Substanz der ersten verdickenden Substanzen ist Gellan Gum, das unter der Bezeichnung Kelcogel® CG-HA vertrieben wird (CP Kelco France, Levallois-Perret, Frankreich). Ebenfalls bevorzugt ist Carbomer.

Vorteilhafte Konzentrationen der Gruppe der ersten verdickenden Substanzen liegen im Bereich von 0,1 bis 0,5 Gew.-%, insbesondere 0,15 bis 0,3 Gew.-%, bezogen auf das Gesamtgewicht des kosmetischen Präparates.

Bevorzugte Substanzen der Gruppe der zweiten verdickenden Substanzen sind Carboxymethylcellulose, Veegum ® als Magnesium-Aluminium-Silicat (INCI:
Magnesium Aluminium Silicate) und ein Pflanzenextraktgemisch mit der INCI-Bezeichnung Glycerine & Leuconostoc/Radish Root Ferment Filtrate & Lonicera Japonica Flower Extract & Lonicera Caprifolium Flower Extract & Populus Tremuloides Bark Extract & Gluconolactone, das unter der Bezeichnung NataPres® von Univar Europa vertrieben wird und das besonders bevorzugt ist.

Es wurde gefunden, daß obwohl NataPres® nicht als Verdicker bekannt ist, es zusammen mit der ersten und dritten Gruppe verdickenden Substanzen zu einer überraschend vorteilhaften Verdickungswirkung beiträgt.

Das genannte Pflanzenextraktgemisch NataPres® ist zugleich ein ausgezeichnetes Konservierungsmittel.

Vorteilhafte Konzentrationen der Substanzen der zweiten Gruppe liegen im Bereich von 1,5 bis 3 Gew.-%, insbesondere 2,0 bis 2,8 Gew.-%, bezogen auf das Gesamtgewicht des kosmetischen Präparates.

Die dritte Gruppe verdickenden Substanzen wird von Stärken gebildet. Als Stärke können bekannte Stärken wie Reisstärke, Maisstärke, Weizenstärke sowie modifizierte Stärken verwendet werden.

Eine bevorzugte Stärke ist eine modifizierte Maisstärke mit der INCI-Bezeichnung Distarch Phosphate, die unter der Bezeichnung Corn P04 PH"B" von Dr. Hauser GmbH, Garmisch-Partenkirchen, Deutschland erhältlich ist. Diese modifizierte Stärke ist ein Distärkephosphatester auf Basis von Maisstärke, der quervernetzt ist.

Vorteilhafte Konzentrationen der Substanzen der dritten Gruppe mit verdickender Wirkung liegen im Bereich von 3 bis 6 Gew.-%, insbesondere 3,5 - 4,8 Gew.-%, bezogen auf das Gesamtgewicht des kosmetischen Präparates.

Die drei Gruppen verdickender Substanzen bilden in dem wäßrigen Gemisch einen besonderen Verdickerkomplex, durch dessen Zusammenwirken die homogene Verteilung der Q-10-Liposomen und der Fluorcarbonliposomen und deren stabiles Halten in der Gelstruktur unterstützt wird. Dabei liegt das Verhältnis von erster Gruppe : zweiter Gruppe : dritter Gruppe verdickender Substanzen vorteilhaft im Bereich von 1 : 8-15 : 15-30. Die Einstellung dieses Verhältnisses beeinflußt die Länge des Zeitraumes ohne Entmischungserscheinungen und führt damit zu vorteilhaften Wirkungen.

Der in diesem Zusammenhang benutzte Begriff "Verdickerkomplex" ist kein Komplex im rein chemischen Sinne, sondern ein Gemisch verschiedener Substanzen mit Gelbildungs- und/oder Verdickungseigenschaften, deren Zusammenwirken eine bisher nicht bekannte und nicht vorhersehbare Wirkung oder einen speziellen Aspekt dieser Eigenschaften zeigt. Die Wirkung des Verdickerkomplexes geht damit über die Summe der Einzelwirkungen hinaus, da mit allen Komponenten der drei Gruppen mit verdickender Wirkung in einzelner Anwendung die gefundenen Vorteile nicht erreicht werden.

Das wäßrige gelartige Gemisch enthält weiterhin eine zweite in Liposomen verkapselte Substanz auf Basis von Fluorcarbonen. Die gebildeten Liposomen, die die Fluorcarbone einschließen, werden vorteilhaft aus Wasser und Glycosphingolipiden gebildet. Als Fluorcarbone können eingesetzt werden Perfluordecalin, Bis-F(butyl)ethen, F-Tripropylamin, F-Tributylamin, F-Dihexylether oder F-Hexan. Besonders bevorzugt ist Perfluordecalin. Ein bekanntes Produkt ist Cerasome ® O₂ Energy (Rovi GmbH, Schlüchtern, Deutschland) mit der INCI-Zusammensetzung Water & Perfluorodecaline & Propylene Glycol & Glycerine & Jojoba Oil & Sucrose Palmitate & Glycosphingolipids & Xanthan Gum & Preservative.

Der Gehalt dieser zweiten verkapselten Substanz liegt im Bereich von 3 bis 4 Gew.-%, bezogen auf das Gesamtgewicht des kosmetischen Präparates.

Diese zweite verkapselte Substanz bildet infolge der kritischen Löslichkeitstemperatur der Fluorcarbone für Sauerstoff bereits ein Depot von molekularem Sauerstoff in der Haut und dient der beschleunigten Zellerneuerung. Neben einer Verbesserung der Hautfeuchtigkeit wird auch die Prolinhydroxylierung zur Bildung von kollagenem Gewebe unterstützt und erhalten.

Bevorzugt wird die zweite in Liposomen verkapselte Substanz jedoch noch zusätzlich zuvor mit reinem Sauerstoff (O₂) bei 20 - 25°C behandelt, da die Fluorcarbone in der Lage sind, zusätzlich Sauerstoff aufzunehmen.

In dem erfindungsgemäßen wäßrigen gelartigen Gemisch werden infolge von dessen spezieller Zusammensetzung die O₂-angereicherten Liposomen besonders stabil gehalten, so daß die unvermeidlichen O₂-Verluste erst sehr spät auftreten und dann auch noch geringer sind als zu erwarten (s. Beispiel 4). Dies ist ein weiterer Vorteil des erfindungsgemäßen Präparates. Das Produkt ist damit bekannten Zusammensetzungen, die Fluorcarbone und ein Verdickungsmittel enthalten, deutlich überlegen.

Eine wirksame Ergänzung zu den O₂-Liposomen sind zugesetzte Radikalfänger wie z. B. Vitamin E und dessen Derivate, vorzugsweise Tocopherolacetat, -palmitat, - succinat, -propionat, -oleat, -sorbat oder-linolat, die auch im Gemisch eingesetzt werden können.

Ein weiterer vorteilhafter Bestandteil des wäßrigen gelartigen Gemisches als Sebumregulierendes Agens ist Dipotassium Glycyrrhizate mit einem Anteil von 0,4 - 0,9 Gew.-%, bezogen auf das Gesamtgewicht des kosmetischen Präparates.

In einer bevorzugten Ausführungsform der Erfindung liegt die erste verkapselte Substanz in einem ersten Container vor, das gelartige Gemisch in einem zweiten Container, und die beiden Container sind durch eine Öffnung miteinander verbunden, und der erste Container ist in seinem Volumen verkleinerbar.

Der erste Container kann dabei durch Druck von außen verkleinert werden, wobei zugleich die bisher verschlossene Öffnung zwischen beiden Containern geöffnet wird, und die erste verkapselte Substanz mit dem wäßrigen gelartigen Gemisch in Kontakt kommt, und infolge der etwa gleichen Viskosität ein Mischvorgang eintritt, der durch Einwirkung von außen (Schütteln, Kippen der Container) beschleunigt werden kann.

Vorzugsweise ist die erste verkapselte Substanz eingefärbt, um den Mischvorgang sichtbar nachvollziehbar zu machen.

Unter dem Begriff "erste verkapselte Substanz" wird eine Emulsion mit Liposomen verstanden, die wenigstens Wasser, Lecithin und Coenzym Q10 (INCI: Ubiquinone) enthält. Ein entsprechendes Handelsprodukt ist z. B. PhytoSolve® 4068, das in Liposomen eingeschlossenes Q-10 in einer Menge von 10 g / 100 g enthält (Lipoid GmbH, Ludwigshafen, Deutschland) und mit der INCI-Bezeichnung Glycerine & Water & Ubiquinone & Lecithine & Caprylic/Capric Triglycerides & Tocopheryl Acetate erhältlich ist.

Besonders vorteilhafte Konzentrationen der ersten in Liposomen verkapselten Substanz liegen im Bereich von 24 bis 36 Gew-%, vorzugsweise 28-34 Gew-%, insbesondere 31-34 Gew.-%. Ein weiterer vorteilhafter Bereich liegt bei 26 - 29 Gew.-%.

Die Viskositäten der ersten in Liposomen verkapselten (oder eingeschlossenen) Substanz und des wäßrigen gelartigen Gemisches liegen jeweils bei 1000 - 3000 mPa·s, wobei beide Komponenten nicht die gleiche Viskosität haben müssen. Die Viskosität wird nach der Brookfield-Methode gemessen bei 25°C mit einer Tellerspindel RV3.

Wenn die Viskosität auf höher als 3000 mPa·s ansteigt, ist eine homogene Vermischung der beiden Komponenten des Zweikomponenten-Präparates nicht mehr gewährleistet und nur noch schwierig zu erreichen.

Die Erfindung betrifft auch die Verwendung eines kosmetischen Zweikomponenten-Präparates, bestehend aus 20 bis 40 Gew-% einer ersten in Liposomen verkapselten Substanz auf Basis von Ubichinon-10 und 60 bis 80 Gew-% eines wäßrigen, gelartigen Gemisches, wobei das wäßrige gelartige Gemisch
a) eine erste Gruppe verdickender Substanzen, ausgewählt unter Gellan Gum, Carbomer, Xanthan Gum, Guar Gum und Gemischen davon,
b) eine zweite Gruppe verdickender Substanzen, ausgewählt unter Gummiarabicum, Tragenth, verschiedenen Cellulosen, Pektin, Magnesium-Aluminium-Silicaten, einem Pflanzenextraktgemisch aus Leuconostoc/Radish Root Ferment Filtrate, Lonicera Japonica Flower Extract, Lonicera Caprifolium Flower Extract und Populus Tremuloides Bark Extrakt, sowie Gemischen davon,
c) eine dritte Gruppe verdickender Substanzen, ausgewählt unter Stärken, wobei das Verhältnis der ersten, zweiten und dritten Gruppe verdickender Substanzen im Bereich von 1 : 6 - 40 : 6 - 60 liegt,
d) einen einwertigen C₂-C₄-Alkohol und
e) eine zweite in Liposomen verkapselten Substanz auf Basis von Fluorcarbonen umfaßt,
und die Viskosität nach Brookfield der ersten verkapselten Substanz und des wäßrigen gelartigen Gemisches jeweils im Bereich von 1000 bis 3000 mPa·s liegt, gemessen bei 25°C, Tellerspindel RV3,
und wobei die erste verkapselte Substanz in einem von dem gelartigen Gemisch getrennten Container vorliegt, und wobei die Konzentrationsangaben auf das Gesamtgewicht des Präparats bezogen sind,
in einem Verfahren zur topischen Behandlung von alternder Haut, bei welchem der Inhalt der beiden Container vor der Anwendung miteinander vermischt wird.

Die Vermischung erfolgt vorzugsweise durch Verschütteln der beiden Containerinhalte durch den Anwender.

Das gleichzeitige Vorhandensein von Q-10-Liposomen und O₂-tragenden Liposomen führt überraschend zu einer sehr schnellen positiven Zustandsänderung von alternder Haut. Es stellt sich schon nach wenigen Tagen (2 - 4) ein deutlich verbessertes Hautbild ein.

Die Herstellung eines Verdickerkomplexes aus den drei Gruppen verdickender Substanzen erfolgt erfindungsgemäß durch Verrühren der ersten Gruppe verdickender Substanzen mit Wasser bei 50-70°C. Daran schließt sich ein Abkühlvorgang auf 30-45°C an und die Zugabe der zweiten und dritten Gruppe verdickender Substanzen und weiterer kosmetischer Hilfsstoffe und anschließendes Homogenisieren bei höchstens 1500 U/min für 10 bis 15 Minuten zur Bildung des Verdickerkomplexes. Dann erfolgt ein Abkühlen auf 20-30°C, Zugabe der zweiten verkapselten Substanz und homogenes Verrühren bei 200-300 U/min und Lagerung des Präparates bei 12-20°C.

Weiterhin ist ein kosmetisches Zweikomponenten-Präparat mit der obigen Definition zur Verwendung zur topischen Behandlung von alternder Haut offenbart.

Des Weiteren ist ein kosmetisches Zweikomponenten-Präparat mit der obigen Definition zur Verwendung zur topischen Behandlung von Haut mit zusätzlich in der zweiten verkapselten Substanz gebundenem Sauerstoff offenbart. Der Sauerstoff wird überraschend wahrscheinlich durch den Verdickerkomplex stabilisiert und kann dadurch längere Zeit seine Wirkung auf der Haut entfalten.

Offenbart ist auch die Verwendung des genannten kosmetischen Zweikomponenten-Präparates in einem Verfahren zum stabilisierten Einsatz von Sauerstoff, der in der zweiten verkapselten Substanz zusätzlich gelöst vorliegt, für den Einsatz bei alternder Haut.

Die Erfindung soll nachstehend durch Beispiele näher erläutert werden. Alle Konzentrationsangaben sind in Gewichtsprozent.

### Beispiel 1 Repair Gel I

**Teil 1**

| | |
|---|---|
| PhytoSolve® 4068¹ | 27 % |
| Teil 2 | 73 % |
| bestehend aus | |
| Gellan Gum | 0,1 % |
| NataPres® | 2,3 % |
| Distarch Phosphate | 3,2% |
| Cerasome® O₂ Energy² | 3,3% |
| Glycerine | 4,0% |
| Dipotassium Glycyrrhizate | 0,5% |
| Ethanol | 6,0% |
| Tocopheryl Acetate | 1,0% |
| Water | q.s. ad 73 % |

| | |
|---|---|
| ¹ enthält 10 % Ubiquinone; INCI: Glycerine & Water & Ubiquinone & Lecithin & Caprylic Capric Triglycerides & Tocopheryl Acetate ² mit O₂ angereichert; INCI: Water & Perfluorodecaline & Propylene Glycol & Glycerine & Simmondsia Chinensis Seed Oil & Sucrose Palmitate & Glycosphingolipids & Xanthan Gum & Phenoxyethanol & Benzoic Acid & Dehydroacetic Acid & Ethylhexylglycerine & Polyaminopropyl Biguanide. | |

### Arbeitsanweisung:

Der Teil 2 wird wie folgt hergestellt.

In das auf 70°C erhitzte Wasser wird Gellan Gum eingerührt und damit ein Quellen in Gang gesetzt. Nach dem Abkühlen auf etwa 45°C werden NataPres® und die Stärke zugegeben und unter Rühren die weiteren Bestandteile mit Ausnahme von Ethanol und Cerasome®. Nach dem weiteren Abkühlen auf eine Temperatur im Bereich von 20 bis 30°C wird unter Rühren der Alkohol hinzugegeben und schließlich die für 15 Minuten, wie oben angegeben, zusätzlich mit O₂ behandelten Cerasome® O₂ Energy. Die separate O₂-Behandlung erfolgt 15 Minuten mit reinem Sauerstoffgas bei 25°C und einem Partialdruck von 420 mbar. Das Gemisch Teil 2 wird weitere 3 Minuten vorsichtig bei 200-300 U/min gerührt.

Teil 1 wird bei Umgebungstemperatur (20 - 30°C) in eine Kammer eines Containers gefüllt. Teil 2 wird in die separate zweite Kammer des Containers gefüllt. Die Kammern sind durch eine Trennwand mit einer noch verschlossenen Öffnung voneinander getrennt. Beide Kammern werden nach außen verschlossen. Der Container wird bei 12 - 20°C gelagert.

Im Anwendungsfall wird die Öffnung in der Trennwand der beiden Kammern durchstoßen, so daß eine Vermischung beider Kammerinhalte z. B. durch Schütteln erfolgen kann. Das vermischte Produkt wird vorteilhaft im Kühlschrank gelagert.

### Beispiel 2 Repair Gel II

**Teil 1**

| | |
|---|---|
| PhytoSolve® 4068¹ | 31,7 % |
| Farbstoff | 0,3 % |
| Teil 2 | 68 % |
| bestehend aus | |
| Gellan Gum | 0,25 % |
| NataPres® | 2,7 % |
| Distarch Phosphate | 4,3% |
| Cerasome® O₂ Energy² | 3,6% |
| Glycerine | 4,2% |
| Dipotassium Glycyrrhizate | 0,4% |
| Ethanol | 6,3% |
| Tocopheryl Acetate | 1,0% |
| Water | q.s. ad 68 % |

| | |
|---|---|
| ¹ enthält 10 % Ubiquinone ² mit O₂ angereichert | |

Bezüglich der Herstellung von Teil 2 und das Einbringen in einen Container wird wie in Beispiel 1 verfahren. Teil 1 wird zuvor noch mit einem Farbstoff vermischt.

### Beispiel 3

Es wird wie im Beispiel 2 gearbeitet. Anstelle von 0,25 % Gellan Gum werden 0,30 % Carbomer eingesetzt, und anstellen von 2,7 % NataPres® werden 2,5% Carboxymethylcellulose eingesetzt.

### Beispiel 4

Es wird wie im Beispiel 1 gearbeitet. Anstelle von 2,3 % NataPres® werden 1,0 % Pektin und 2,0 % Veegum ® eingesetzt sowie zusätzlich noch 0,1 % Carbomer.

### Beispiel 5 Vergleichsbeispiel 1

Es wird ein kosmetisches Präparat gemäß Beispiel 1 hergestellt, wobei die Teile 1 und 2 unter Rühren bei 800 U/min für 15 Minuten sorgfältig miteinander vermischt werden. Das Gemisch wird in eine transparente Plastikflasche luftdicht abgefüllt und das konfektionierte Produkt bei 15°C im Kühlschrank für 6 Monate gelagert. Für dieses, als Gel "A" bezeichnete Produkt werden Sichtkontrollen nach 14 Tagen, 1 Monat, 2, 3, 4 und 6 und 8 Monaten durchgeführt.

Das zweite kosmetische Präparat gemäß Beispiel 1 wird wie im Beispiel 1 angegeben in zwei separaten Teilen 1 und 2 hergestellt und separat in einem Behälter mit zwei Kammern abgefüllt, die durch eine noch verschlossene Öffnung miteinander verbunden sind. Der transparente Zweikammerbehälter aus dem gleichen Plastikmaterial wie beim Gel "A" wird ebenfalls luftdicht verschlossen und in gleicher Weise wie oben gelagert. Das Produkt wird als Gel "B" bezeichnet und ebenso kontrolliert wie Gel "A". Nach 6 Monaten werden die Teile 1 und 2 von Gel "B" mittels Durchstoßen der Öffnung und Schütteln vermischt.

Die Ergebnisse sind in Tabelle 1 aufgeführt.

**Tabelle 1**

| | Gel "A" | Gel "B" |
|---|---|---|
| | | Teil 1 und Teil 2 |
| nach 14 Tagen | unverändert | beide unverändert |
| nach 1 Monat | unverändert | beide unverändert |
| nach 2 Monaten | Beginn Schichtenbildung | beide unverändert |
| nach 3 Monaten | ausgeprägtere zwei Schichten | beide unverändert |
| nach 4 Monaten | ausgeprägtere Schichtenbildung | beide unverändert |
| nach 6 Monaten | ausgeprägtere Schichtenbildung | erst beide unverändert, nach Mischen dann Gemischstruktur (weißlich statt transluzent) |
| Nach 8 Monaten | Ausgeprägtere Schichtenbildung | Unveränderte Gemischstruktur |

Aus der Tabelle 1 ist ersichtlich, daß der Separationsvorgang bei dem Gel "A" bereits nach 2 Monaten Lagerung eingesetzt hat und im wesentlichen nach 3 Monaten abgeschlossen ist. Dagegen ist das erfindungsgemäße Gel "B" bis zur Vereinigung der Teile 1 und 2, die jeweils transluzent sind, unverändert. Auch 2 Monate danach (nach 8 Monaten) ist die anfängliche Gemischstruktur, die nach 6 Monaten hergestellt worden war, noch unverändert und ohne Schichtenbildung erhalten.

### Beispiel 6 Vergleichsbeispiel 2

Es wird der Einfluß auf den O₂-Gehalt in der zweiten in Liposomen verkapselten Substanz am Beispiel der Cerasome® O₂ Energy untersucht.

8g Cerasome® O₂ Energy werden 15 Minuten mit reinem Sauerstoffgas bei 22°C und einem Partialdruck von 420 mbar durchperlt.

Von diesen modifizierten 8g werden danach 4g abgenommen, 4 Minuten bei 100 U/min bei 25°C gerührt und dann in einem verschlossenen Glasbehälter bei 8°C im Kühlschrank für 2 Monate gelagert (Probe 2A).

Die anderen 4 g modifizierten Cerasome® O₂ Energy werden in das Gemisch von Teil 2 gemäß Beispiel 1 eingebracht und ebenfalls dabei insgesamt 4 Minuten bei 200 U/min bei 25°C gerührt. Danach werden die Teile 1 und 2 gemäß Beispiel 1 mit dem Rührstab vermischt und in einen verschlossenen Glasbehälter bei 8°C im Kühlschrank für 2 Monate gelagert (Probe 2B).

Nach Ablauf der Lagerzeit werden die O₂-Gehalte von Probe 2A und 2B gemessen. Die Messung erfolgt mit einem Oxi 3000 (WTW GmbH, Weilheim, Deutschland) in Vol.-% O₂ der Anfangskonzentration.

| | |
|---|---|
| Probe 2A | 4 % |
| Probe 2B | 18 % |

## Patentansprüche

1. Kosmetisches Zweikomponenten-Präparat, **dadurch gekennzeichnet, daß** es 20 bis 40 Gew-% einer ersten in Liposomen verkapselten Substanz auf Basis von Ubichinon-10 und
60 bis 80 Gew-% eines wäßrigen, gelartigen Gemisches umfaßt, bestehend aus
a) einer ersten Gruppe verdickender Substanzen, ausgewählt unter Gellan Gum, Carbomer, Xanthan Gum, Guar Gum und Gemischen davon,
b) einer zweiten Gruppe verdickender Substanzen, ausgewählt unter Gummiarabicum, Tragenth, verschiedenen Cellulosen, Pektin, Magnesium-Aluminium-Silicaten, einem Pflanzenextraktgemisch aus Leuconostoc/Radish Root Ferment Filtrate, Lonicera Japonica Flower Extract, Lonicera Caprifolium Flower Extract und Populus Tremuloides Bark Extrakt, sowie Gemischen davon,
c) einer dritten Gruppe verdickender Substanzen, ausgewählt unter Stärken, wobei das Verhältnis der ersten, zweiten und dritten Gruppe verdickender Substanzen im Bereich von 1 : 6 - 40 : 6 - 60 liegt,
d) einem einwertigen C₂-C₄-Alkohol und
e) einer zweiten in Liposomen verkapselten Substanz auf Basis von Fluorcarbonen,
und die Viskosität nach Brookfield der ersten verkapselten Substanz und des wäßrigen gelartigen Gemisches jeweils im Bereich von 1000 bis 3000 mPa·s liegt, gemessen bei 25°C, Tellerspindel RV3,
und wobei die erste verkapselte Substanz in einem von dem gelartigen Gemisch getrennten ersten Container vorliegt und das gelartige Gemisch in einem zweiten Container, und wobei die Konzentrationsangaben auf das Gesamtgewicht des Präparats bezogen sind,
hergestellt durch Verrühren der ersten Gruppe verdickender Substanzen mit Wasser bei 50-70°C, danach Abkühlen auf 30-45°C und Zugabe der zweiten und dritten Gruppe verdickender Substanzen und weiterer kosmetischer Hilfsstoffe und anschließendes Homogenisieren bei höchstens 1500 U/min für 10 bis 15 Minuten zur Bildung eines Verdickerkomplexes, Abkühlen des Verdickerkomplexes auf 20-30°C, Zugabe des Alkohols und der zweiten verkapselten Substanz und homogenes Verrühren bei 200-300 U/min und Lagerung des Präparates bei 12-20°C.

2. Kosmetisches Präparat nach Anspruch 1, **dadurch gekennzeichnet, dass** die erste verkapselte Substanz in einem ersten Container vorliegt, das gelartige Gemisch in einem zweiten Container vorliegt, und die beiden Container durch eine Öffnung miteinander verbunden sind, und der erste Container in seinem Volumen verkleinerbar ist.

3. Kosmetisches Präparat nach Anspruch 1 oder 2, **dadurch gekennzeichnet, dass** Ubichinon-10 in Liposomen aus Lecithin und Wasser mit einer Konzentration von 10 Gew-% vorliegt, bezogen auf das Gewicht der Liposomen.

4. Kosmetisches Präparat nach einem der Ansprüche 1-3, **dadurch gekennzeichnet, dass** die Konzentration der ersten verkapselten Substanz im Bereich von 24 bis 36 Gew-% liegt, vorzugsweise 28-34 Gew-%, insbesondere 31-34 Gew-%.

5. Kosmetisches Präparat nach einem der Ansprüche 1-4, **dadurch gekennzeichnet, daß** die erste verdickende Substanz Gellan Gum ist.

6. Kosmetisches Präparat nach einem der Ansprüche 1-5, **dadurch gekennzeichnet, daß** die zweite verdickende Substanz ein Pflanzenextraktgemisch ist mit der INCI-Bezeichnung Glycerine & Leuconostoc/Radish Root Ferment Filtrate & Lonicera Japonica Flower Extract & Lonicera Caprifolium Flower Extract & Populus Tremuloides Bark Extract & Gluconolactone.

7. Kosmetisches Präparat nach einem der Ansprüche 1-6, **dadurch gekennzeichnet, daß** die dritte verdickende Substanz ein quervernetzter Maisstärke-Ester ist, insbesondere Distarch Phosphate.

8. Kosmetisches Präparat nach einem der Ansprüche 1-7, **dadurch gekennzeichnet, daß** das Verhältnis von erster: zweiter: dritter verdickender Substanz im Bereich von 1 : 8-15 : 15-30 liegt.

9. Kosmetisches Präparat nach einem der Ansprüche 1-8, **dadurch gekennzeichnet, dass** die zweite verkapselte Substanz in Liposomen aus Glycosphingolipiden und Wasser vorliegt und zusätzlich gelösten Sauerstoff enthält.

10. Kosmetisches Präparat nach einem der Ansprüche 1-9, **dadurch gekennzeichnet, dass** der Anteil der zweiten verkapselten Substanz im Bereich von 3 bis 4 Gew-% liegt, bezogen auf das Gesamtgewicht des Präparates.

11. Kosmetisches Präparat nach einem der Ansprüche 1-10, **dadurch gekennzeichnet, dass** der Anteil des Alkohols im Bereich von 5 bis 7 Gew-% liegt, bezogen auf das Gesamtgewicht des Präparates.

12. Verwendung eines kosmetischen Zweikomponenten-Präparates nach einem der Ansprüche 1 bis 11 in einem nicht-therapeutischen Verfahren zur topischen Behandlung von alternder Haut.

## Claims

1. A cosmetic two-component preparation, **characterized in that** it comprises 20 to 40% by weight of a first substance based on coenzyme Q₁₀, encapsulated in liposomes, and 60 to 80% by weight of an aqueous, gel-like mixture, consisting of
a) a first group of thickening agents selected from Gellan Gum, Carbomer, Xanthan Gum, Guar Gum and mixtures thereof,
b) a second group of thickening agents selected from rubber arabicum, gum tragacanth, various celluloses, pectin, magnesium-aluminium-silicates, a plant extract mixture made of Leuconostoc/Radish Root Ferment Filtrate, Lonicera Japonica Flower Extract, Lonicera Caprifolium Flower Extract and Populus Tremuloides Bark Extract, as well as mixtures thereof,
c) a third group of thickening agents selected from starches, wherein the ratio of the first, second and third group of thickening agents is in the range of 1 : 6 - 40 : 6 - 60,
d) a monovalent C₂-C₄ alcohol, and
e) a second substance based on fluorocarbons, encapsulated in liposomes,
and the Brookfield viscosity of the first encapsulated substance and the aqueous gel-like mixture is, in each case, in the range of 1000 to 3000 mPa·s,
measured at 25°C using plate spindle RV3,
and wherein the first encapsulated substance is present in a first container separated from the gel-like mixture and the gel-like mixture is present in a second container, and wherein the concentrations indicated are based on the total weight of the preparation,
produced by mixing the first group of thickening agents with water at 50-70°C,
then cooling to 30-45°C and adding the second and third group of thickening agents and additional cosmetic auxiliaries and subsequent homogenizing at a maximum of 1500 rpm for 10 to 15 minutes in order to form a thickener complex,
cooling the thickener complex to 20-30°C, adding the alcohol and the second encapsulated substance and homogeneously mixing at 200-300 rpm and storing the preparation at 12-20°C.

2. The cosmetic preparation according to Claim 1, **characterized in that** the first encapsulated substance is present in a first container, the gel-like mixture is present in a second container, and the two containers are connected to one another by an opening, and the volume of the first container can be reduced.

3. The cosmetic preparation according to Claim 1 or 2, **characterized in that** coenzyme Q10 is present in liposomes comprising lecithin and water having a concentration of 10% by weight, based on the weight of the liposomes.

4. The cosmetic preparation according to any one of Claims 1-3, **characterized in that** the concentration of the first encapsulated substance is in the region of 24 to 36% by weight, preferably 28-34% by weight, in particular 31-34% by weight.

5. The cosmetic preparation according to any one of Claims 1-4, **characterized in that** the first thickening agent is gellan gum.

6. The cosmetic preparation according to any one of Claims 1-5, **characterized in that** the second thickening agent is a plant extract mixture with the INCI name Glycerine & Leuconostoc/Radish Root Ferment Filtrate & Lonicera Japonica Flower Extract & Lonicera Caprifolium Flower Extract & Populus Tremuloides Bark Extract & Gluconolactone.

7. The cosmetic preparation according to any one of Claims 1-6, **characterized in that** the third thickening agent is a cross-linked corn starch ester, in particular distarch phosphate.

8. The cosmetic preparation according to any one of Claims 1-7, **characterized in that** the ratio of first: second : third thickening agent is in the region of 1 : 8-15 : 15-30.

9. The cosmetic preparation according to any one of Claims 1-8, **characterized in that** the second encapsulated substance is present in liposomes comprising glycosphingolipids and water and, in addition, dissolved oxygen.

10. The cosmetic preparation according to any one of Claims 1-9, **characterized in that** the proportion of the second encapsulated substance is in the region of 3 to 4% by weight, based on the total weight of the preparation.

11. The cosmetic preparation according to any one of Claims 1-10, **characterized in that** the proportion of the alcohol is in the region of 5 to 7% by weight, based on the total weight of the preparation.

12. Use of a cosmetic two-component preparation according to any one of Claims 1 to 11 in a non-therapeutic method for topically treating ageing skin.

## Revendications

1. Préparation cosmétique bicomposant, **caractérisée en ce qu'**elle comprend 20 à 40 % massique d'une première substance encapsulée dans des liposomes à base d'ubiquinone 10, et 60 à 80 % massique d'un mélange aqueux de type gel, se composant de :
a) un premier groupe de substances épaississante, sélectionnées parmi : Gellan Gum, Carbomer, Xanthan Gum, Guar Gum, et de mélanges de celles-ci.
b) un deuxième groupe de substances épaississantes sélectionnées parmi : gomme arabique, traganthe, diverses celluloses, pectine, silicates d'aluminium de magnésium, un mélange d'extraits de plantes de Leuconostoc/Radish Root Ferment Filtrate, Lonicera Japonica Flower Extract, Lonicera Caprifolium Flower Extract et Populus Tremuloides Bark Extract, ainsi que des mélanges de celles-ci,
c) un troisième groupe de substances épaississantes, sélectionnées parmi des amidons, le rapport des premier, deuxième et troisième groupes de substances épaississantes se trouvant dans la plage 1 : 6 - 40 : 6 - 60.
d) un alcool C₂-C₄ monovalent et
e) une deuxième substance encapsulée dans des liposomes à base de fluorocarbones,
et la viscosité selon Brookfield de la première substance encapsulée et du mélange aqueux de type gel se trouvant respectivement dans la plage de 1 000 à 3 000 mPa•s, mesurée à 25 °C, broche à disque RV3,
et la première substance encapsulée se trouvant dans un premier conteneur séparé du mélange de type gel, et le mélange de type gel dans un deuxième conteneur, et les indications de concentration étant rapportées au poids total de la préparation,
fabriquée par mélange du premier groupe de substances épaississantes avec de l'eau à 50-70 °C, suivi par un refroidissement à 30-45 °C et ajout des deuxième et troisième groupes de substances épaississantes et d'autres agents auxiliaires cosmétiques et homogénéisation subséquente à 1 500 t/min au maximum pendant 10 à 15 minutes pour la formation d'un complexe épaississant, refroidissement du complexe épaississant à 20-30 °C, ajout de l'alcool et de la deuxième substance encapsulée et mélange homogène à 200-300 t/min et stockage de la préparation à 12-20 °C.

2. Préparation cosmétique selon la revendication 1, **caractérisée en ce que** la première substance encapsulée se trouve dans un premier conteneur, le mélange de type gel se trouve dans un deuxième conteneur et les deux conteneurs sont reliés l'un à l'autre par une ouverture, et le premier conteneur peut être réduit relativement à son volume.

3. Préparation cosmétique selon la revendication 1 ou 2, **caractérisée en ce que** l'ubiquinone 10 se trouve dans des liposomes de lécithine et d'eau avec une concentration de 10 % massique par rapport au poids des liposomes.

4. Préparation cosmétique selon l'une quelconque des revendications 1 à 3, **caractérisée en ce que** la concentration de la première substance encapsulée se trouve dans la plage de 24 à 36 % massique, de préférence 28-34 % massique, en particulier 31-34 % massique.

5. Préparation cosmétique selon l'une quelconque des revendications 1 à 4, **caractérisée en ce que** la première substance épaississante est Gellan Gum.

6. Préparation cosmétique selon l'une quelconque des revendications 1 à 5, **caractérisée en ce que** la deuxième substance épaississante est un mélange d'extraits de plantes avec la désignation INCI Glycerine & Leuconostoc/Radish Root Ferment Filtrate & Lonicera Japonica Flower Extract & Lonicera Caprifolium Flower Extract & Populus Tremuloides Bark Extract & Gluconolactone.

7. Préparation cosmétique selon l'une quelconque des revendications 1 à 6, **caractérisée en ce que** la troisième substance épaississante est un ester d'amidon de maïs réticulé, en particulier Distarch Phosphate.

8. Préparation cosmétique selon l'une quelconque des revendications 1 à 7, **caractérisée en ce que** le rapport entre les première : deuxième : troisième substances épaississantes se trouve dans la plage de 1 : 8-15 : 15-30.

9. Préparation cosmétique selon l'une quelconque des revendications 1 à 8, **caractérisée en ce que** la deuxième substance encapsulée se trouve dans des liposomes de glycosphingolipides et d'eau et contient en outre de l'oxygène dissous.

10. Préparation cosmétique selon l'une quelconque des revendications 1 à 9, **caractérisée en ce que** la part de la deuxième substance encapsulée se trouve dans la plage de 3 à 4 % massique par rapport au poids total de la préparation.

11. Préparation cosmétique selon l'une quelconque des revendications 1 à 10, **caractérisée en ce que** la part de l'alcool se trouve dans la plage de 5 à 7 % massique par rapport au poids total de la préparation.

12. Utilisation d'une préparation bicomposant cosmétique selon l'une quelconque des revendications 1 à 11 dans un procédé non thérapeutique pour le traitement topique de peau vieillissante.
